(19) Europäisches Patentamt
European Patent Office
Office européen des brevets

(11) Publication number: **0 493 682 A2**

# (12) EUROPEAN PATENT APPLICATION

(21) Application number: **91120426.1**

(51) Int. Cl.[5]: **A61K 31/52**, A61K 37/02

(22) Date of filing: **28.11.91**

(30) Priority: **30.11.90 US 620479**

(43) Date of publication of application:
**08.07.92 Bulletin 92/28**

(84) Designated Contracting States:
**AT BE CH DE DK ES FR GB GR IT LI LU NL SE**

(71) Applicant: **HOECHST-ROUSSEL PHARMACEUTICALS INCORPORATED**
**Route 202-206 North**
**Somerville New Jersey 08876(US)**

(72) Inventor: **Bianco, James A.**
**2920 Alki Avenue**
**Seattle, Washington 98116(US)**
Inventor: **Singer, Jack W.**
**3515 East Spring Street**
**Seattle, Washington 98122(US)**
Inventor: **Novick, William J., Jr.**
**R.D. Nr. 2, Bartles Road**
**Lebanon, NJ 08833(US)**

(74) Representative: **Becker, Heinrich Karl Engelbert, Dr. et al**
**HOECHST AKTIENGESELLSCHAFT Central Patent Department P.O. Box 80 03 20**
**W-6230 Frankfurt am Main 80(DE)**

## (54) Use of xanthines for the preparation of a medicament effective for inhibiting the effects of allograft reaction in humans.

(57) A family of compounds effective in inhibiting the effects of allograft reaction, such as graft versus host reaction (GVHR), in a human is comprised of 7-(oxoalkyl) 1,3-dialkyl xanthines of the formula

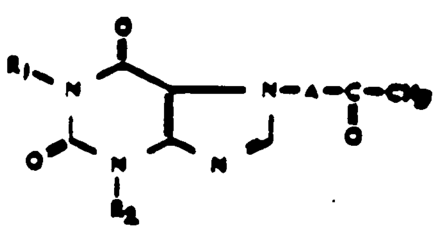

(I)

in which $R_1$ and $R_2$ are the same or different and are selected from the group consisting of straight-chain or branched alkyl radicals with 2 to 6 carbon atoms, cyclohexyl, alkoxyalkyl and hydroxyalkyl radicals, and A represents a hydrocarbon radical with up to 4 carbon atoms which can be substituted by a methyl group. Another family of effective compounds is comprised of compounds of the formula

EP 0 493 682 A2

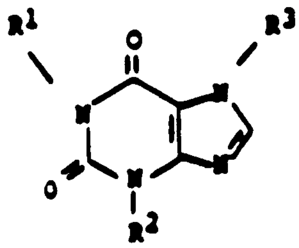

(II)

wherein at least one of $R_1$ and $R_3$ is either

a) a branched hydroxyalkyl group of the formula

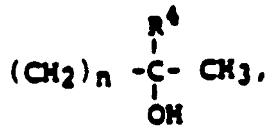

in which $R^4$ stands for an alkyl group with 1 to 3 carbon atoms and n stands for a whole number from 2 to 5, the other $R^1$ or $R^3$ group that may optionally be present stands for a hydrogen atom or an aliphatic hydrocarbon group $R^5$ with up to 6 carbon atoms, whose carbon chain may be interrupted by up to 2 oxygen atoms or may be substituted with a hydroxy or oxo group, or

b) an oxoallyl group of the formula

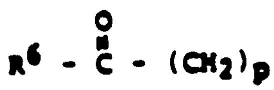

wherein $R^6$ is $C_1$-$C_6$ and p is 2, 3 or 4, the remaining $R^1$ or $R^3$ being as defined above, and $R^2$ is an alkyl group $C_1$-$C_4$;The inhibition of GVHR is indicated in human transplant recipients.

MLC-PENTOXIFYLLINE RESPONSE

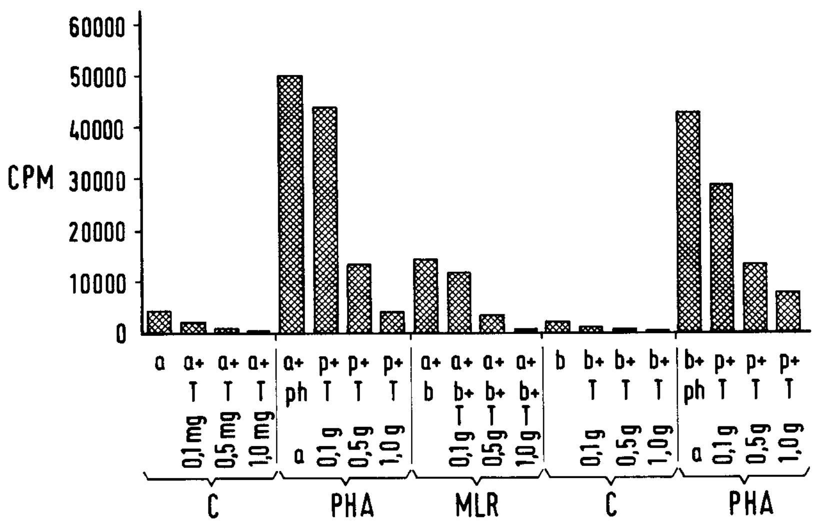

## BACKGROUND OF THE INVENTION

This invention relates to the use of xanthines to inhibit allograft reaction in humans. In one embodiment, this invention relates to the use of xanthines to inhibit the effects associated with graft versus host reaction (GVHR) in humans. In another embodiment, this invention relates to a method of using certain xanthines to inhibit the manifestations of GVHR in humans following transplantation therapy.

Transplantation or grafting involves the transfer of living cells, tissues, or organs from one part of the body to another or from one individual to another. The fate of a graft is determined by the genetic relationship between the donor and host. Each species has a group of histocompatibility genes coding for histocompatibility antigens that determine compatibility or incompatibility of tissue transplants. The antigens make up the major histocompatibility complex (MHC), which appears to be of overwhelming importance in determining the fate of transplants.

Generally, grafts exchanged between animals that do not differ in histocompatibility genes are accepted, whereas those transplanted between individuals differing in histocompatibility genes are rejected. For example, rejection will occur if the recipient of a graft is missing a histocompatibility gene present in the donor of the graft. The rejection is brought about by allograft reaction, which is a specific immune response against histocompatibility (transplantation) antigens.

The allograft reaction is a great source of frustration to the transplant surgeon; the reaction can negate the benefits of even the most skillful work. The allograft reaction involves an attack by lymphocytes of a host against the graft as if the graft was a foreign invader. This response by the host can result in transplant failure.

In most situations, the allograft reaction is a one-way process: The host reacts against the graft ("host versus graft reaction" or "HVGR"). Often, however, the graft contains lymphocytes, and in this instance an immunological response of the graft against the host can occur. In some cases this "graft versus host reaction" or "GVHR" remains unnoticed because the HVGR prevails and all the transplanted cells are rejected before they can harm the host. When the host is unable to mount the HVGR reaction, the consequences of GVHR become clearly visible.

GVHR is a complex process, which is not fully understood. The general reaction sequence probably involves the inoculation of donor lymphocytes into an immuno-incompetent host. The donor lymphocytes undergo blast transformation and eventually small lymphocytes are formed. These small lymphocytes have a killer capacity capable of carrying out the destruction of host tissue. Alter the cells are disseminated and proliferate throughout the organs and tissues of the host, GVHR results.

The proliferation of the donor lymphocytes and the damage the resulting killer cells inflict on the host tissue set in motion a complex response from the host. The most prominent features of this response are inflammation at the site of donor cell proliferation, increased phagocytic activity, and increased proliferation of the reticuloendothelial and lymphoid cells in the spleen and liver. The clinical reaction is marked by enlargement of a variety of hematopoietic and lymphopoietic tissues, skin rashes, bowel disturbances, and other allergic-type manifestations. In the meantime, the donor derived cytotoxic cells begin to attack the lymphoid tissue of the host thereby destroying large populations of cells. These effects weaken the resistance of the host to pathogens, and as a result, secondary complications begin to appear. In the most severe cases of GVHR, the recipient eventually dies.

The allograft reaction is immunologically mediated. Thus, efforts to prevent its development have involved the use of immunosuppressive therapy. Methotrexate, cyclophosphamide, and cyclosporine have been found to be useful in marrow transplantation, but acute GVHR remains a serious problem. In fact, about one-third of the patients who develop moderate to severe GVHR will die with this complication.

Although MHC disparities are the most potent stimulators of GVHR, there are other histocompatibility loci capable of eliciting this reaction. It is for this reason that GVHR remains a major impediment to bone marrow transplantation. Some centers have restricted bone marrow transplantation to situations in which an identical sibling is available. Given the highly complex nature of the histocompatibility system, it seems unlikely that one will be able to obtain complete matching among unrelated individuals on a routine basis. Therefore, bone marrow transplantation among some unrelated individuals must be postponed until better means for specific immunotherapy are available. In the case of liver transplantation, waiting for a good match of the MHC is currently not practical because of the precarious medical condition of most liver transplant recipients prior to surgery.

It should be apparent that there exists a need in the art for the clinical management of transplant patients, including maintaining the process of graft acceptance or rejection and providing adequate treatment. Because the existing treatments and means for preventing allograft reaction, such as HVGR and acute GVHD are unsatisfactory, new approaches in preventing or treating allograft rejection must be found,

since allograft rejection is major cause of mortality and morbidity in transplant patients.

## SUMMARY OF THE INVENTION

This invention aids in fulfilling these needs in the art by providing a method of inhibiting allograft reaction, such as host versus graft reaction (HVGR) and graft versus host reaction (GVHR), in humans. More particularly, this invention provides a method of inhibiting the allograft reaction in a human by administering to the human at least one 7-(oxoalkyl) 1,3-dialkyl xanthine, other than denbufylline, of the formula:

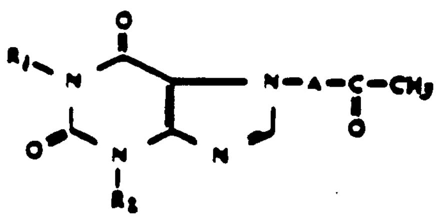

(I)

in which

$R_1$ and $R_2$ are the same or different and are independently selected from the group consisting of straight-chain or branched-chain alkyl radicals with 2 to 6 carbon atoms, cyclohexyl, straight chain or branched chain alkoxyalkyl, and hydroxyalkyl radicals; and

A is a hydrocarbon radical with up to 4 carbon atoms which can be substituted by a methyl group.

The xanthine of formula (I) is employed in an amount that is effective in inhibiting allograft reaction. In a preferred embodiment of the invention, the xanthine of formula (I) is administered in an amount that is effective in inhibiting the effects of GVHR in a human.

This invention also provides a method of inhibiting allograft reaction in a human comprising administering to the human a compound of the formula:

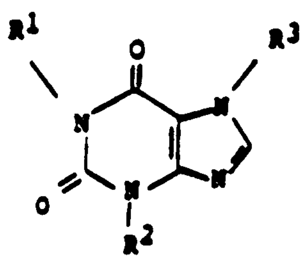

(II)

wherein at least one of $R^1$ and $R^3$ is either

(a) a branched hydroxyalkyl group of the formula

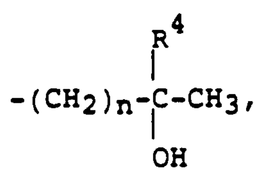

with a tertiary alcohol function, in which $R^4$ stands for an alkyl group with 1 to 3 carbon atoms and n stands for a whole number from 2 to 5, the other $R^1$ or $R^3$ group that may optionally be present stands for a hydrogen atom or an aliphatic hydrocarbon group $R^5$ with up to 6 carbon atoms, whose carbon chain may be interrupted by up to 2 oxygen atoms or may be substituted with a hydroxy or oxo group, or

(b) at least one of $R^1$ or $R^3$ is an oxoallyl group of the formula

$$R^6-\overset{O}{\overset{\|}{C}}-(CH_2)_p-,$$

wherein $R^6$ is $C_1$-$C_6$ alkyl, and p = 2, 3 or 4; the other $R^1$ or $R^3$ being defined as above and $R^2$ represents an alkyl group with 1 to 4 carbon atoms.

The xanthine of formula (II) is employed in an amount that is effective in inhibiting allograft reaction. In a preferred embodiment of the invention, the xanthine of formula (II) is administered in an amount that is effective in inhibiting the effects of GVHR in a human. The well known pharmaceutical pentoxifylline is an example of a compound within the general formula (II). Pentoxifylline ("PTX") is commercially available under the trademark Trental® in the form of tablets for oral administration. Although this compound has been used for time as a pharmaceutical to improve the flow properties of blood (clinical trials in 1971), it has not been reported effective as an inhibitor of allograft reaction, such as GVHR.

## BRIEF DESCRIPTION OF THE DRAWINGS

This invention will be more fully understood by reference to the Figure, which depicts the effects of pentoxifylline on the uptake of $^3$H-thymidine by cultured lymphocytes.

## DETAILED DESCRIPTION OF PREFERRED EMBODIMENT

Certain xanthines are employed according to this invention to inhibit the effects associated with responses of T lymphocytes in human subjects. The nature of the biological processes involved in this invention will initially be described. This will be followed by a detailed description of the xanthines and methods for preparing the xanthines. The results obtained by *in vivo* and *in vitro* testing will follow.

### 1. The Allograft Reaction and Allograft Rejection

This invention makes it possible to inhibit the effects of allograft reaction in human subjects. More particularly, xanthines are employed according to this invention to modulate the immune responses to allografts where untreated rejection would otherwise lead to graft loss. Acute graft rejection is predominantly a cell-mediated immune response, and the xanthines when employed according to the invention suppress activation or functioning of several types of effector cells (other than effector inflammatory cells). That is, the xanthines suppress activation of lymphocytes, namely T lymphocytes and B lymphocytes, of the host and the donor. Suppression of lymphocyte activation by the xanthines is achieved to a sufficient extent to allow acquired tolerance to an allograft to be established in the graft recipient, with or without other immunosuppressive therapy. This results in stable and prolonged engraftment.

The xanthines suppress activation of one or more lymphocytes selected from the group consisting of T helper cells, T cytotoxic cells, T suppressor cells, natural killer cells, killer cells responsible for antibody dependent cell-mediated cytotoxicity, T delayed hypersensitivity cells, B lymphocytes, and subsets of each of these cell types. Suppression of lymphocyte activation by the xanthines can be demonstrated even in the absence of leukocyte-derived cytokines, such as tumor necrosis factor (TNF).

Allograft reaction can be manifested as host versus graft reaction (HVGR) or as graft versus host reaction (GVHR) in human subjects. HVGR can lead to graft failure or rejection due to host resistance or persistent host immunity. Host cells may impair the engraftment of donor-derived tissue or the proliferation and differentiation of donor-derived cells.

GVHR can occur when three conditions are fulfilled. First of all, GVHR requires that the host possesses major histocompatibility complex (MHC) antigens, which are substantially absent from the graft. HLA typing can be used to minimize the occurrence of histocompatibility antigens capable of inducing GVHR, but in most cases compatibility for the entire HLA system cannot be realized and transplantation must be carried out with some degree of mismatch. GVHR increases in severity with the incompatibility between donor and recipient.

Secondly, GVHR requires that the graft contains immunologically competent lymphocytes. The graft lymphocytes typically originate from lymph node cells, peripheral blood leukocytes, spleen cells, bone marrow cells, and thymocytes. T lymphocytes apparently initiate GVHR, although other cells may be present and become involved. Immature T lymphocytes are generally unable to initiate GVHR.

Thirdly, GVHR requires that the recipient host is immunologically incompetent or immunosuppressed or

deficient to the extent that the host is unable to reject the graft. While GVHR typically occurs in immunologically incompetent or suppressed recipient hosts, it will be understood that GVHR can also occur in immunologically competent recipients in which the effects of GVHR are not overshadowed by a more powerful HVGR.

The transfer of cells, tissue, or organ from one individual to another leading to an allograft reaction is typically associated with transplantation therapy. Thus, for example, allograft reaction can be associated with bone marrow grafts, kidney transplantation, heart transplantation, skin grafting, cornea transplantation, as well as the transplantation of other organs and tissue, such as liver, lung, spleen, pancreas, intestine, tendon, bone, cartilage, blood vessels, and larynx. GVHR frequently accompanies the transfer of immunologically competent cells from one individual to another, such as the transplantation of lymphoid tissues, such as suspensions prepared by homogenization of a donor organ or cells, for example bone marrow, lymph nodes, spleen, thymus, or liver, and inoculated into the recipient host.

This invention is useful for inhibiting the effects of GVHR and HVGR accompanying all types of transplantation therapy. Thus, for example, this invention is useful for treating HVGR and GVHR accompanying grafts placed in the same anatomical position normally occupied by the transplanted tissue (orthotopic) or those placed in an unnatural position (heterotropic). Similarly, the HVGR and GVHR can accompany grafts taken from and placed on the same individual (autografts), grafts transplanted between genetically identical individuals (isografts), grafts transplanted between genetically different individuals of the same species (allografts), and grafts transplanted between individuals of two different species (xenografts).

In a preferred embodiment of this invention, the xanthines are administered to a clinical marrow transplant recipient. Use of the xanthines according to the invention enables the inhibition of the effects of GVHR and GVHD accompanying marrow transplantation. Thus, use of this invention is preferred for patients in which it is desired to establish normal marrow function with transplanted cells, such as patients with severe combined immunodeficiency disease or aplastic anemia, and patients receiving chemotherapy and radiation as aggressive treatment for leukemia and other malignancies. The transplanted marrow cells can be from a matched donor or preserved cells from the patient from whom the cells were previously removed and stored. Untreated marrow cells can be employed, or the marrow cells can be treated, such as by lymphocyte purging to deplete the marrow of cells associated with the occurrence of GVHD. Methods to remove T cells include treatment with antithymocyte globulin, anti-T-cell monoclonal antibodies, complement-mediated lysis, lectin agglutinin sedimentation, sheep erythrocyte rosetting, counterflow centrifugal elutriation, immunobeads linked with anti-T-cell monoclonal antibodies, or magnetic beads linked to anti-T-cell monoclonal antibodies. Although T-cell depletion of the marrow inoculum prevents acute GVHD, there may be an increased incidence of graft rejection or leukemic relapses, or a decrease in graft-versus-leukemia effect. It has been found that T-cell depletion of marrow inoculum is not required when the xanthines are employed according to the invention.

GVHR has also been observed in patients other than those undergoing transplantation therapy. For example, severely immunodepressed patients may inadvertently become engrafted by foreign lymphoid cells during routine blood transfusion. It will be understood that this invention is useful for inhibiting allograft reaction and the effects of GVHR and GVHD occurring in non-transplant patients.

GVHR is manifested as GVHD in the human, which can be diagnosed by clinical indications and chemical techniques. Among the clinically observed effects of GVHD are fever, malaise, hyperthermia, and pain in the area of a transplant, hypertension, weight loss, and impairment of spleen function. In the case of bone marrow transplantation, GVHD is indicated by skin rash and blistering, severe nausea, vomiting, diarrhea, gastrointestinal bleeding, or impairment of liver function. Allograft rejection following liver transplantation is characterized by bile duct destruction and hepatocellular necrosis.

The effects of GVHR and GVHD can also be determined by biochemical techniques, such as measurement of enzyme levels and waste or degradation products of biological processes in the patient. For example, GVHD can be ascertained by the measurement of plasma creatinine levels in kidney transplantation, or the levels of alkaline phosphatase, transaminase, and bilirubin in the case of liver transplants.

A xanthine or a mixture of the xanthines can be administered to a human subject in need of therapy in an amount sufficient to inhibit one or more of the foregoing effects of allograft reaction. Thus, for example, one or more of the clinical indications or biochemical changes manifesting GVHR or GVHD can be reduced in incidence or severity, or alleviated, prevented, or controlled by administration of the xanthines according to this invention. While mixtures of xanthines can be employed, no advantage has been observed in the use of mixtures.

When the xanthines are employed in conjunction with transplant therapy in a patient, the xanthines can be administered before, during, or after transplantation. The xanthines are generally orally or intravenously

administered to the patient. In a preferred embodiment of this invention, the xanthines are intravenously administered to the patient immediately following transplantation, and then orally administered to a transplant recipient as post-transplant immunosuppressive therapy.

Inhibition of GVHR according to this invention can be demonstrated *in vitro*. More particularly, cocultivation of leukocytes from unrelated individuals leads to blast transformation and proliferation similar to that observed upon addition of mitogens to T-cells. The basis of this reaction appears to be the activation of T-cells from one individual by cell surface markers of another. Co-culturing of blood leukocytes from two individuals is referred to as "mixed leukocyte culture" or "MLC" and the reaction as "mixed leukocyte reaction" or "MLR". Co-culturing of cells obtained from lymphoid organs is designated "mixed lymphocyte culture" or "mixed lymphocyte reaction". Since even in the mixed leukocyte reaction, the participating cells are peripheral blood T lymphocytes, there is generally substantially no difference between the two types of culture. Thus, these expressions are used interchangeably herein.

The property of stimulating an MLR is a function of disparity at the MHC of stimulating cells and responder cells. Since disparity at the MHC is also a major cause of allograft reaction, MLR is utilized in this invention as the *in vitro* analog of allograft reaction in the human. More particularly, MLC can be employed in this invention as an *in vitro* assay for determining the preferred xanthines and the amount thereof to employ in a particular clinical setting. A xanthine that gives a strong response in inhibiting human MLR is preferred for use in inhibiting GVHR and GVHD according to this invention.

MLR can be carried out using conventional techniques. Cultured cells for the MLR can be derived from peripheral blood, spleen, lymph nodes, or thymus. Cell number can vary by several orders of magnitude. The stimulating cells can be inactivated by irradiation or treatment with an antibiotic to disrupt DNA replication. The culture medium can be supplemented, if necessary, by serum proteins of autologous, syngeneic, allogeneic, or xenogeneic origin. Cultivation can be carried out over a period of several days. The degree of stimulation of responder cells by stimulating cells can be determined from the uptake rate of $^{3}H$-thymidine or radiolabeled amino acids or by counting blasts and dividing cells. If a radiolabel is employed, it can be added to the culture several hours before harvesting.

The effect of the xanthines on GVHR according to the invention was determined in MLR. More particularly, six independent dose-response experiments using five different donors were performed using both response to PHA and the MLR. Cells were exposed to pentoxifylline or diluent for six days. $^{3}H$ thymidine was added for three hours and the cells were harvested and counted using a scintillation counter. The data was normalized by setting the control CPM at 100% and the test substance (pentoxifylline) CPM was determined as the mean of the ratio of CPM to the control counts. The results are shown in the Figure. Pentoxifylline suppressed both the MLR and the response to PHA. At 1mM, pentoxifylline suppressed the PHA response to 53 ± 9% of the control and the MLR to 42 ± 6% of control. The viability of cells was 100% using trypan blue dye-exclusion.

This procedure was repeated using the compound. 7-ethoxymethyl-1-(5-hydroxy-5-methylhexyl-3-methylxanthine. This compound was similar to pentoxifylline in suppressing both the MLR and the response to PHA. This compound at 100 $\mu$g/ml suppressed the PHA response to 41 ± 5% of control and the MLR to 39 ± 3% of control. The concentration of this compound was less than one-half of that of pentoxifylline and this compound had at least as much activity as pentoxifylline under the test conditions.

The procedure was once again repeated using the compound 1-(5-hydroxy-5-methylhexyl)-3-methylxanthine and denbufylline (i.e. 7-acetonyl-1,3-dibutylxanthine). The first compound had minimal activity and denbufylline appeared inactive at the concentrations tested.

When cells, tissue, and organs are transplanted across an MHC incompatibility, allograft reaction can occur. Incompatibilities at minor histocompatibility loci can usually be overcome by minimal immunosuppressive regimens, whereas inhibition of rejection due to incompatibilities at the major locus requires much more aggressive immunosuppressive therapy. It will be understood that this invention can be employed alone or in combination with such minimal or aggressive immunosuppressive therapies in the patient.

More particularly, immunosuppressive agents can be employed with the xanthines according to this invention to lower immune response of the host and to at least retard the allograft reaction. That is, the xanthines can be employed according to the invention, either before, after, or simultaneously with the use of chemical immunosuppressive agents, such as alkylating agents, antimetabolites, corticosteroids, or antibiotics; physical immunosuppressive agents, such as ionizing radiation; immune reagents, such as antilymphocytic serum or antilymphocytic globulins; or by the administration of antigens or polyclonal or monoclonal antibodies for specific suppression.

More particularly, in the case of bone marrow transplantation, the xanthines can be administered before, during, or after the administration of immunosuppressive agents, such as cyclophosphamide, methotrexate, cyclosporin A, prednisone, methylprednisone, antithymocyte globulin, monoclonal antibodies reactive with

human lymphocyte subpopulations, or combinations of these therapies. These immunosuppressants are employed according to the regimens usually used for immunosuppression in marrow transplantation to inhibit GVHD.

Similarly, in the case of renal transplantation, the xanthines can be employed according to the invention in conjunction with well known immunosuppressive therapies used for this type of transplantation. Thus, for example, the xanthines can be employed in the post-operative management of renal transplant by administering the xanthines before, during, or after administration of corticosteroids, such as predhisone and methyl prednisone; cyclosporin A; azathioprine; antilymphocytic serum or globulin; monoclonal antibodies reactive with human lymphocyte subpopulations; or combinations of these therapies. Once again, these immunosuppressants are employed according to the regimens usually used for immunosuppression in renal transplant recipients.

The practice of this invention can be accompanied by the administration of hematopoietic growth factors to the allograft recipient to enhance engraftment or to reduce the incidence, severity, or duration of clinical manifestations of allograft reaction. In a preferred embodiment of the invention, therapy of bone marrow transplant recipients with the xanthines is accompanied by the administration of granulocytemacrophage colony stimulating factor (GM-CSF). The GM-CSF appears to accelerate hematologic recovery following bone marrow transplantation.

The invention can also be employed in conjunction with the conventional pre-transplant treatments utilized to reduce the risk of allograft rejection. The preparative regimen should eradicate all disease in the recipient, immunosuppress the patient adequately to permit sustained engraftment in the allogeneic setting, and be minimally toxic. Patients typically receive pretransplant drug therapy to suppress the host immune system with or without whole body or limited field radiation in a single dose or fractionated over several days. Thus, for example, a patient receiving whole body radiation to reduce the risk of transplant rejection can be treated with xanthines according to this invention to reduce the manifestations of GVHD.

Thin invention can be employed to treat allograft reaction, such as HVGR or GVHR, in a human subject, or the invention can be employed as a prophylactic for HVGR or GVHR. This invention makes it possible to improve a patient's condition and sense of well being by inhibiting acute GVHD and may reduce the incidence of chronic GVHD. An increase in patient survival is contemplated.

## 2. Administration of Xanthines to Human Patients

Effective amounts of the xanthines can be administered to a subject by any one of various methods, for example, orally as capsules or tablets, or parenterally, such as by intravenous or intramuscular injection, in the form of sterile solutions. The xanthines, while effective themselves, can be formulated and administered in the form of their pharmaceutically acceptable addition salts for purposes of stability, convenience of crystallization, increased solubility, and the like.

Preferred pharmaceutically acceptable addition salts include salts of mineral acids, for example, hydrochloric acid, sulfuric acid, nitric acid, and the like; salts of monobasic carboxylic acids, such as, for example, acetic acid, propionic acid, and the like; salts of dibasic carboxylic acids, such as, maleic acid, fumaric acid, oxalic acid, and the like; and salts of tribasic carboxylic acids, such as, carboxysuccinic acid, citric acid, and the like.

The xanthines can be administered orally, for example, with an inert diluent or with an edible carrier. They can be enclosed in gelatin capsules or compressed into tablets. For the purpose of oral therapeutic administration, the compounds can be incorporated with excipients and used in the form of tablets, troches, capsules, elixirs, suspensions, syrups, wafers, chewing gums, and the like. These preparations should contain at least 0.5% of active compound, but the amount can be varied depending upon the particular form and can conveniently be between 4.0% to about 70% of the weight of the unit. The amount of xanthine in such compositions is such that a suitable dosage will be obtained. Preferred compositions and preparations according to the present invention are prepared so that an oral dosage unit form contains between about 1.0 mgs and about 400 mgs of active compound.

Tablets, pills, capsules, troches, and the like can contain the following ingredients: a binder, such as microcrystalline cellulose, gum tragacanth or gelatin; an excipient, such as starch or lactose; a disintegrating agent, such as alginic acid, Primolgel, corn starch, and the like; a lubricant, such as magnesium sterate or Sterotes; a glidant, such as colloidal silicon dioxide; a sweetening agent, such as sucrose or saccharin; or flavoring agent, such as peppermint, methyl salicylate, or orange flavoring. When the dosage unit form is a capsule, it can contain, in addition to material of the above type, a liquid carrier, such as a fatty oil.

Other dosage unit forms can contain other materials that modify the physical form of the dosage unit, for example, as coatings. Thus, tablets or pills can be coated with sugar, shellac, or other enteric coating

agents. A syrup can contain, in addition to the active compounds, sucrose as a sweetener, and preservatives, dyes, colorings, and flavors. Materials used in preparing these compositions should be pharmaceutically pure and non-toxic in the amounts used.

For purposes of parenteral therapeutic administration, the xanthines can be incorporated into a solution or suspension. These preparations should contain at least 0.1% of the aforesaid compound, but may be varied between 0.5% and about 50% of the weight thereof. The amount of active compound in such compositions is such that a suitable dosage will be obtained. Preferred compositions and preparations according to the present invention are prepared so that a parenteral dosage unit contains between 0.5 mg to 100 mgs of the active compound.

Solutions or suspensions of the xanthines can also include the following components: a sterile diluent, such as water for injection, saline solution, fixed oils, polyethylene glycols, glycerine, propylene glycol or other synthetic solvents; antibacterial agents, such as benzyl alcohol or methyl parabens; antioxidants, such as ascorbic acid or sodium bisulfite; chelating agents, such as ethylenediaminetetraacetic acid; buffers, such as acetates, citrates, or phosphates; and agents for the adjustment of tonicity, such as sodium chloride or dextrose. The parenteral preparation can be enclosed in ampoules, disposable syringes, or multiple dose vials made of glass or plastic.

While dosage values will vary, good results are achieved when the xanthines of formula (I) or formula (II) are administered to a subject requiring such treatment as an effective oral, parenteral, or intravenous dose of from 0.1 to 25 mg/kg of body weight per day. A particularly preferred regimen for use in bone marrow transplant therapy comprises the intravenous administration of PTX in aqueous saline solution (100 mg/5 ml) at 1/2 mg/kg/hour continuously for 20 days, followed by 2,000 mg/day oral dosage to day 100 post-transplant. It is to be understood, however, that for any particular subject, specific dosage regimens should be adjusted to the individual need and the professional judgment of the person administering or supervising the administration of the xanthines. It is to be further understood that the dosages set forth herein are exemplary only and that they do not, to any extent, limit the scope or practice of the invention.

3. Description and Preparation of Xanthines of Formula (I)

One group of xanthines that can be employed in this invention has the following formula:

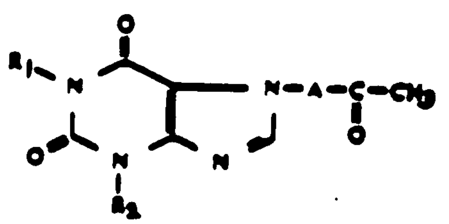

(I)

The substituents $R_1$ and $R_2$ in formula (I) are the same or different and are independently selected from the group consisting of straight-chain or branched alkyl radicals with 2 to 6 carbon atoms, cyclohexyl, alkoxyalkyl, and hydroxyalkyl radicals. The substituent A represents a hydrocarbon radical with up to 4 carbon atoms, which can be substituted by a methyl group.

A number of compounds of formula (I) can be employed in this invention. For example, the xanthines of formula (I) can be substituted by alkyl groups or by alkoxy or hydroxyalkyl groups. Suitable alkyl groups include branched and straight chain groups, such as ethyl, propyl, isopropyl, butyl, sec-butyl, tert-butyl, amyl, hexyl, and the like. Alkoxy substituted alkyl groups include branched and straight chain groups containing from 2 to 6 carbon atoms in the combined alkoxy and alkyl groups, including methoxymethyl, amyloxymethyl, methoxyethyl, butoxyethyl, propoxypropyl, and the like. Hydroxyalkyl groups are those containing from 1 to 6 carbon atoms, such as hydroxymethyl, hydroxyethyl, hydroxypropyl, hydroxyhexyl, and the like.

The hydrocarbon groups represented by A in formula (I) are divalent saturated aliphatic hydrocarbon groups, i.e., methylene, ethylene, trimethylene and tetramethylene, which can be substituted on the carbon adjacent the carbonyl group with methyl. Such methyl-substituted groups include ethylidine, 1,2-propylene, and 1,3-butylene groups.

It will be understood that the method of this invention can be practiced with compounds that change *in vivo* into one of the aforementioned xanthines of formula (I), as well as compounds that produce

metabolites *in vivo* similar to the metabolites formed from the aforementioned xanthines of formula (I).

A compound within formula (I) that has been found to be not effective for inhibiting the effects of GVHR is denbufylline. This compound, which is also referred to herein in abbreviated form as "DBOPX", has the following formula:

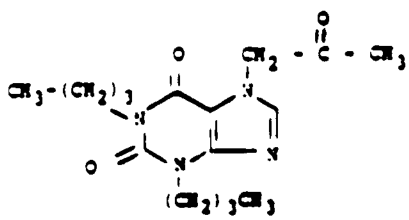

(III)

"DBOPX"

The ability of compound (III) to inhibit the effects of GVHR as evidenced by MLR has not been demonstrated *in vitro*.

The compounds of formula (I) employed in this invention can be synthesized using known techniques. For example, the compounds can be prepared at elevated temperature, optionally in the presence of a solvent, by reacting correspondingly substituted 1,3-dialkyl xanthines of the formula

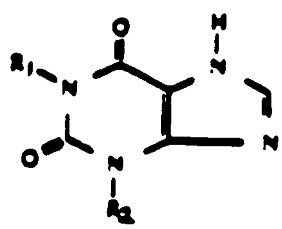

(III)

in which $R_1$ and $R_2$ are as defined above, with $\alpha$, $\beta$-unsaturated methyl ketones corresponding to the formula

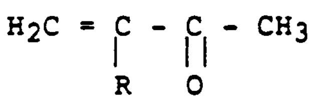

(IV)

The substituent R in formula (IV) represents hydrogen or a methyl group. The reaction can be conducted in an alkaline medium.

An alternative method of preparation involves reacting alkali metal salts of 1,3-dialkyl xanthine derivatives of general formula II, in which $R_1$ and $R_2$ are as defined above, with oxoalkyl halides corresponding to the formula

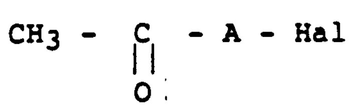

(V)

in which A is as defined above, and Hal represents a halogen atom, preferably chlorine or bromine.

These reactions are preferably carried out at temperatures in the range from 40° to 80°C, optionally under elevated or reduced pressure, but usually at atmospheric pressure. The individual starting compounds can be employed either in stoichiometric quantities or in excess. The alkali salts in the alternative method of preparation can either be prepared beforehand or in the reaction itself.

Suitable solvents for use in the reactions are water-miscible compounds, preferably lower alcohols, such as methanol, propanol, isopropanol, and various butanols; also acetone; pyridine; triethylamine; polyhydric alcohols, such as ethylene glycol and ethylene glycol monomethyl or monethyl ether.

The compounds of formula (I) are known for their marked effect in increasing blood flow through skeletal muscle and by their low toxicity. A more detailed description of the compounds employed in this invention and methods of preparing the compounds are contained in U.S. Patent 4,242,345, the entire disclosure of which is relied upon and incorporated by reference herein.

4. Description and Preparation of Xanthines of Formula (II)

Inhibition of allograft reaction, and in particular GVHR, can also be achieved by administering to a human patient a xanthine of the formula:

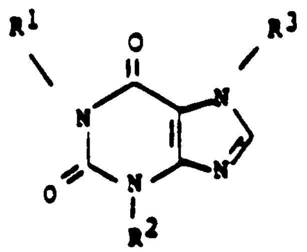

(II)

wherein at least one of $R^1$ and $R^3$ is either

(a) a branched hydroxyalkyl group of the formula

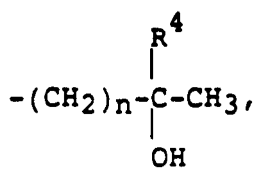

with a tertiary alcohol function, in which $R^4$ stands for an alkyl group with 1 to 3 carbon atoms and n stands for a whole number from 2 to 5, the other $R^1$ or $R^3$ group that may optionally be present stands for a hydrogen atom or an aliphatic hydrocarbon group $R^5$ with up to 6 carbon atoms, whose carbon chain may be interrupted by up to 2 oxygen atoms or may be substituted with a hydroxy or oxo group, or

(b) at least one of $R^1$ or $R^3$ is an oxoallyl group of the formula

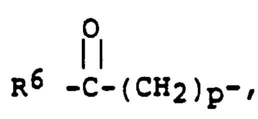

wherein $R^6$ is $C_1$-$C_6$ alkyl, and p = 2, 3 or 4; the other $R^1$ or $R^3$ being defined as above, and $R^2$ represents an alkyl group with 1 to 4 carbon atoms. The xanthine of formula (II) is employed in an amount that is effective in inhibiting allograft reaction, especially GVHR. Among these compounds is the commercially available compound pentoxifylline (Trental®). A host of other compounds within the general formula (II) can be employed for inhibiting GVHR activity. Among these compounds are those identified below.

GVHR-INHIBITING
COMPOUNDS OF FORMULA (II)

| Compound Number | $R_1$ | $R_2$ | $R_3$ |
|---|---|---|---|
| 2 | 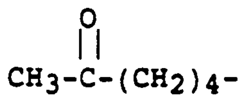 | $-CH_3$ | $-CH_2-CH_2-CH_3$ |
| 3 | 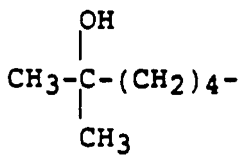 | $-CH_3$ | $-CH_2-CH_2-O-CH_3$ |
| 4 | " | " | $-CH_2-O-(CH_2)_2-O-CH_3$ |
| 5 | " | " | -H |
| 6 | " | " | $-CH_2-CH_2-CH_3$ |
| 7 | " | " | 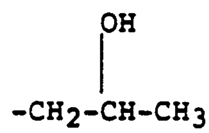 |
| 8 | " | " | 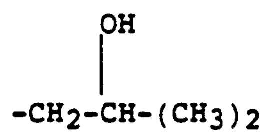 |
| 9 | " | $-CH_2-CH_3$ | $-CH_2-O-CH_2-CH_3$ |
| 10 | " | $-CH_3$ | 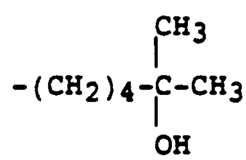 |
| 11 | " | " | $-CH_2-O-CH_2-CH_3$ |

It will be understood that the method of this invention can be practiced with compounds that change *in vivo* into one of the aforementioned xanthines of formula (II), as well as compounds that produce metabolites *in vivo* similar to the metabolites formed from the aforementioned xanthines of formula (II). For example, after oral and intravenous administration, pentoxifylline is almost completely metabolized. The following seven metabolites have been identified in human urine, which is the predominant pathway for excretion of metabolites:

| | |
|---|---|
| Metabolite I | 1-(5-hydroxyhexyl)-3,7-dimethylxanthine |
| Metabolite II | 1-(5,6-dihydroxyhexyl)-3,7-dimethylxanthine |
| Metabolite III | 1-(4,5-dihydroxyhexyl)-3,7-dimethylxanthine |
| Metabolite IV | 1-(4-carboxybutyl)-3,7-dimethylxanthine |
| Metabolite V | 1-(3-carboxypropyl)-3,7-dimethylxanthine |
| Metabolite VI | 1-(5-oxohexyl)-3-methylxanthine |
| Metabolite VII | 1-(5-hydroxyhexyl)-3-methylxanthine. |

Metabolites I and V are the major metabolites. Metabolite V, the main urinary Metabolite, accounts for about 50-60 percent of the administered dose. Only traces of pentoxifylline and Metabolite I are found in urine. The dihydroxy derivatives of pentoxifylline (Metabolites II and III) represent approximately 12 percent and Metabolite IV about 8 percent of the excretion products.

The compounds of formula (II) can be prepared according to the disclosure of U.S. Patent 3,737,433 and Belgium Patent 831,051 (where $R^1/R^3$ are oxoallyl). For the case where at least one of $R^1/R^3$ is a tertiary alcohol reference may be had to International Application PCT/EP86/00401, filed July 8, 1966, claiming German priority of July 8, 1985. This application addresses, as its invention, a variety of embodiments of synthesis routes for the xanthines of formula (II) embraced in the current invention.

An example of one embodiment consists of

a) reacting 3-alkylxanthines of formula (VII)

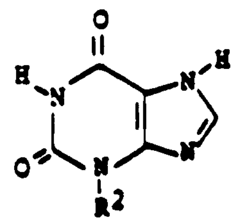

(VII)

in which the $R^3$ represents alkyl with up to 4 carbon atoms, with alkylating agents of formula (VIII)

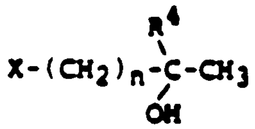

(VIII)

in which X stands for halogen, preferably chlorine, bromine, or iodine, or a sulfonic acid ester group or a phosphoric acid ester group, and wherein $R^4$ and n have the meanings mentioned above, to obtain compounds of formula (IX)

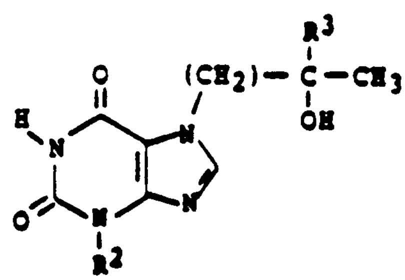

(IX)

with a tertiary hydroxyalkyl group in the position of $R^3$ and hydrogen in the position of $R^1$, and

$a_1$) alkylating this with the same or different alkylating agent of formula (VIII) to obtain compounds pursuant to the invention of formula (X)

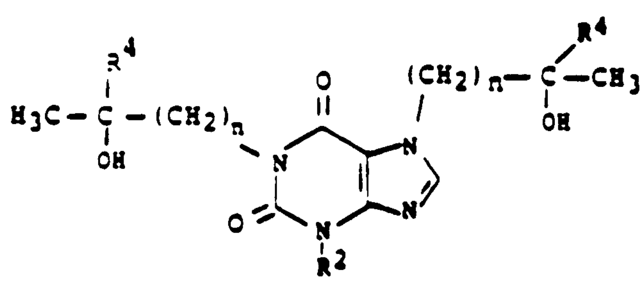

(X)

with two identical or different tertiary hydroxyalkyl groups in the positions of $R^1$ and $R^3$, or $a_2$) converting it with a compound of the formula

$R^5$-X (Xa)

in which X has the meaning given in formula (VIII) and $R^5$ has the meaning indicated above, into compounds of formula (XI)

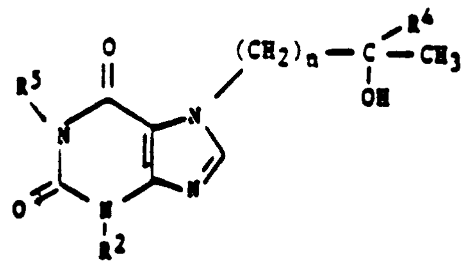

(XI)

in all cases preferably operating in the presence of basic media or using the xanthines in the form of their salts.

Another embodiment consists of

b) substituting 1,3-dialkylated xanthines of formula (XII)

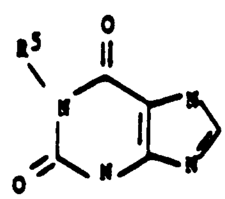

(XII)

in the 7-position, preferably in the presence of basic media or in the form of their salts, by one-step reaction with a compound of formula (VIII), to obtain compounds of formula (XI).

Another embodiment consists of

c) first reacting the 3-alkylxanthines of formula (VII), likewise preferably in the presence of basic media or in the form of their salts, with a compound of the formula

$R^{15}$-X (XIII)

with the formation of 3,7-disubstituted xanthines of formula (XIV)

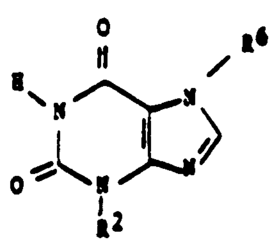

(XIV)

in which $R^{15}$ has the meaning mentioned for $R^5$ or stands for benzyl or diphenylmethyl, and then substituting them in the 1-position, again preferably in the presence of basic media or in the form of their salts, with a compound of formula (VIII). Compounds of formula (XV) are obtained

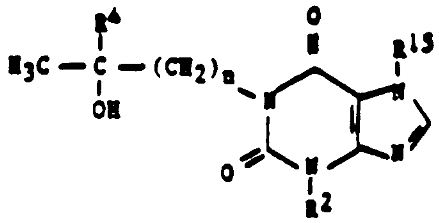

(XV)

in which $R^{15}$ represents a benzyl or diphenylmethyl, and converting the compounds of formula (XV) in which $R^{15}$ represents a benzyl or diphenylmethyl group or an alkoxymethyl or alkoxyalkoxymethyl group, under reducing or hydrolytic conditions, into compounds pursuant to the invention of formula (XVI)

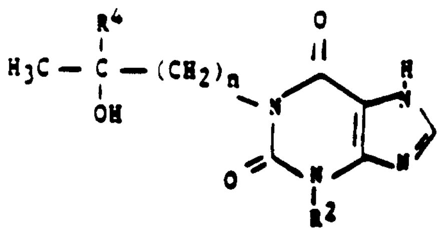

(XVI)

that are subsequently reacted again, if desired, with a compound of formula (VIII) or (Xa) to obtain compounds pursuant to the invention of formula (X) or (XV).

Another embodiment consists of

d) reducing compounds of formula (XI) or (XV) pursuant to the invention in which $R^5$ or $R^{15}$ stands for an oxoalkyl group, with conventional reducing agents for the keto group to obtain the corresponding hydroxyalkylated xanthines pursuant to the invention.

The 3-alkyl- or 1,3-dialkylxanthines of formula (VII) or (XII) used here as starting materials and the "alkylating agents" of formulas (VIII), (Xa), and (XIII) are known for the most part or can be prepared readily by methods disclosed in the literature. Thus, the tertiary alcohols of formula (VIII), for example, can be obtained by organometallic synthesis by reacting the sterically unhindered haloketones of the formula

Hal-$(CH_2)_n$-CO-$CH_3$ (XVII)

in a so-called synthetic reaction with reductive alkylation of the carbonyl group, with alkylmetal compounds $R^4$-M, especially of magnesium, zinc, or lithium, for example in the form of alkylmagnesium halides $R^4$-MgHal (Grignard compounds) or of the alkyllithium compounds $R^4$-Li, under the usual conditions (for example, see Houben-Weyl, Vol. VI/1 a, Part 2 (1980), pp. 928-40, especially pp. 1021 ff. and 1104-1112). In the same way, a reaction of the haloketones with the formula

Hal-$(CH_2)_n$-CO-$R^4$ (XVIII)

with methylmagnesium halides or methyllithium likewise leads to the target.

The hydroxyketones corresponding to the formulas (XVII) and (XVIII) can also be converted smoothly into diols with the alkylmetal compounds in the usual way, either directly or with temporary masking of the hydroxy group, for example by acetal formation with 5,6-dihydro-4H-pyran (for example, see Houben-Weyl, Vol. VI/1 a, Part 2 (1980), pp. 1113-1124), from which compounds of formula (VIII) are formed by selective esterification of the terminal primary hydroxyl groups with sulfonyl or phosphoric halides or anhydrides, advantageously in the presence of basic media.

Other possibilities for the synthesis of the tertiary alcohol derivatives of formula (VIII) consist of the monometallation of ω-chloro-1-bromooalkanes to obtain ω-chloroalkylmetal compounds, (Houben-Weyl, Vol. XIII/2 a (1973), pp. 102 and 319) and their subsequent reaction with the ketones $R^4$-CO-$CH_3$, with the extent of by-product formation from the alkanolates formed as intermediates because of their tendency toward ring closure with the elimination of metal salt being minimized by appropriate temperature control, or of using ω-halo-1-alkanols as starting materials, which are metallated in the usual way, preferably in the form of the

tetrahydropyranyl-(2) ether or after alkanolate formation of the hydroxy group ($MO-CH_2)_n$-Hal) with any desired alkylmetal compound (for example, see Houben-Weyl, Vol. XIII/2 a (1973), p. 113), then reacting them with the ketones $R^4$-CO-$CH_3$ to obtain the diols mentioned in the preceding paragraph (Houben-Weyl, Vol. VI/1 a, Part 2 (1980), p. 1029), and subsequently selectively esterifying the primary hydroxy group with suitable sulfonic or phosphoric acid derivatives.

A convenient access to compounds of formula (VIII) in which $R^4$ represents a methyl group is also available through the reaction of ω-haloalkanoic acid alkyl esters (Hal-$(CH_2)_n$-COO-alkyl) with two equivalents of a methylmetal compound, with the ester reacting through the ketone to produce the tertiary alcohol with the introduction of two methyl groups (Houben-Weyl, Vol. VI/1 a, Part 2 (1980), pp. 1171-1174). In the same way, ω-hydroxy-carboxylic acid esters can be converted into diols with methylmetal compounds with or without protection of the hydroxy group, for example in the form of tetrahydropyranyl-(2) or methoxymethyl ester, or optionally in the form of the lactones as cyclic esters (for example, see Houben-Weyl, Vol. VI/1 a, Part 2 (1980), pp. 1174-1179), from which active alkylating agents of formula (VIII) can in turn be obtained by selective esterification of the primary hydroxyl group with sulfonic or phosphoric halides or anhydrides.

Suitable compounds of formula (VIII) that can be prepared by the methods described above are thus the [(ω-1)-hydroxy-(ω-1)-methyl]butyl, -pentyl, -hexyl, and -heptyl, the [(ω-2)-hydroxy-(ω-2)-methyl]pentyl, -hexyl, -heptyl, and -octyl, and the [(ω-3)-hydroxy-(ω-3)-methyl]hexyl, -heptyl, -octyl, and -nonyl chlorides, bromides, iodides, sulfonates, and phosphates.

Among the compounds of formula $R^5$-X (Xa) or $R^{15}$-X (XIII) suitable for the introduction of $R^5$ into the 1- or 7-position and of $R^{15}$ into the 7-position of the xanthine skeleton, the alkoxymethyl and alkoxyalkoxymethyl derivatives occupy a special position as their halides can indeed be used successfully as reactants, but toxicological problems can arise, at least in large-scale use. For this reason, the use of the corresponding sulfonates is preferred in this special case, which are readily available, for example, by reacting mixed anhydrides of aliphatic carboxylic acids and aliphatic or aromatic sulfonic acids (M.H. Karger et al., J. Org. Chem. 36 (1971), pp. 528-531) with the formaldehyde dialkyl acetals or dialkoxyalkyl acetals in a smooth and nearly quantitative reaction (M.H. Karger et al., J. Amer. Chem. Soc. 91 (1969), pp. 5663/5665:

$$R^7-SO_2-O-CO-(C_1-C_4)Alkyl \quad + \quad R^8-O-CH_2-O-R^8 \quad -(C_1-C_4)Alkyl-CO_2R^8$$

$$\longrightarrow R^7-SO_2-O-CH_2-O-R^8$$

In this equation, $R^7$ represents an aliphatic group such as methyl, ethyl, or trifluoromethyl, or an aromatic group, for example, phenyl, 4-tolyl, or 4-bromophenyl, but preferably methyl or 4-tolyl, and $R^8$ represents an alkyl or alkoxyalkyl group falling under the definition of $R^5$ or $R^{15}$.

The reaction can be carried out either in the substance or in an anhydrous aprotic solvent inert to the reactants at temperatures between -20° and +40°C, preferably between 0° and 20°C. No intermediate isolation of the highly reactive sulfonates, which are sensitive to hydrolysis and thermally labile, is necessary; they are preferably used immediately as crude products for the substitution on the nitrogen of the xanthines, with the usual addition of a basic condensing agent being unnecessary.

The reaction of the mono- or disubstituted xanthine derivatives, (IX), (XVI), (VII), (VIII) or (Xa) or (XIII) is ordinarily done in a distributing agent or solvent inert to the reactants. Practical representatives are especially dipolar, aprotic solvents, for example formamide, dimethylformamide, dimethyl-acetamide, N-methylpyrrolidone, tetramethylurea, hexamethyl-phosphoric triamide, dimethylsulfoxide, acetone, or butanone; however, alcohols such as methanol, ethylene glycol, and their mono- or dialkyl ethers with the alkyl group having 1 to 4 carbon atoms but both together having a maximum of 5 carbon atoms, ethanol, propanol, isopropanol, and the various butanols; hydrocarbons such as benzene, toluene, or xylenes; halogenated hydrocarbons, such as dichloromethane or chloroform; pyridine, and mixtures of the solvents mentioned or their mixtures with water can also be used.

The "alkylation reactions" are suitably carried out in the presence of a basic condensing agent. Examples of materials suitable for this are alkali metal or alkaline earth hydroxides, carbonates, hydrides, alcoholates, and organic bases, such as trialkylamines (for example, triethyl-or tributylamine), quaternary ammonium or phosphonium hydroxides and crosslinked resins with fixed, optionally substituted ammonium

or phosphonium groups. The xanthine derivatives can also be used in the alkylation reaction directly in the form of their separately prepared salts, such as the alkali metal, alkaline earth, or optionally substituted ammonium or phosphonium salts. The mono- and disubstituted xanthine derivatives can also be alkylated either in the presence of the aforementioned inorganic condensing agents or in the form of their alkali metal or alkaline earth salts with the assistance of so-called phase transfer catalysts, for example tertiary amines, quaternary ammonium or phosphonium salts, or crown ethers, preferably in a 2-phase system under the conditions of phase transfer catalysis. Among the suitable phase transfer catalysts that are generally commercially available are tetra ($C_1$-$C_4$)alkyl-and metyltrimethylammonium and -phosphonium salts, methyl-, myristyl-, phenyl-, and benzyltri ($C_1$-$C_4$)alkyl- and cetyltrimethylammonium as well as ($C_1$-$C_{12}$)alkyl- and benzyltriphenylphosphonium salts, with the compounds that have the larger and more symmetrically structured cation generally proving to be the more effective.

The introduction of the groups Ia, $R^5$, and $R^{15}$ by the procedures described above is generally carried out at a reaction temperature between 0 ° C and the boiling point of the particular reaction medium used, preferably between 20 ° and 130 ° , optionally at elevated or reduced pressure, for which the reaction time can amount to less than 1 hour or up to several hours.

The reaction of the 3-alkylxanthines (VIII) to produce the compounds pursuant to the invention of formula (X) requires the introduction of two tertiary hydroxyalkyl groups. Either identical or different substituents can be linked to the xanthine skeleton in succession, or two identical hydroxyalkyl groups can be linked without isolation of intermediates in a single-pot reaction.

The reductive cleavage of the benzyl and diphenylmethyl group from compounds of formula (XV) with the formation of the xanthine atom in the 7-position, is carried out under standard conditions that were developed especially in the framework of the protective group technique in alkaloid and peptide syntheses and can thus be assumed to be widely known. Besides the chemical reduction, particularly of the benzyl compounds with sodium in liquid ammonia (Houben-Weyl, Vol. XI/1 (1957), pp. 974-975), the elimination of the two aforementioned aralkyl groups by catalytic hydrogenolysis using a precious metal catalyst is also especially practical (Houben-Weyl, Vol. XI/1 (1957), pp. 968-971 and Vol. IV//1 c, Part I (1980), pp. 400-404). A lower alcohol is ordinarily used here as the reaction medium (optionally with the addition of formic acid or ammonia), or an aprotic solvent such as dimethylformamide or particularly glacial acetic acid; however, their mixtures with water can also be used. Especially suitable hydrogenation catalysts are palladium black and palladium on activated charcoal or barium sulfate, while other precious metals such as platinum, rhodium, and ruthenium frequently give rise to side reactions because of competitive ring hydrogenation and are therefore only conditionally usable. The hydrogenolysis is preferably carried out at temperatures between 20 ° C and 100 ° C and at atmospheric pressure, or preferably slight excess pressure up to approximately 10 bar, with reaction times of a few minutes to several hours generally being needed.

The 1,3,7-trisubstituted xanthines of formula (XV) that have an alkoxymethyl or alkoxyalkoxymethyl group in the position of $R^{15}$ represent O,N-acetals. Consequently, their substituents in the 7-position can be split off under the usual conditions of acid hydrolysis (cf. Houben-Weyl, Vol. VI/I b (1984), pp. 741-745), with the 7H compounds of formula (XVI) likewise being formed. Examples of preferred groups that can be eliminated hydrolytically are the methoxy, ethoxy, and propoxymethyl groups as well as the methoxyethoxy- and ethoxyethoxymethyl groups. The reaction is advantageously carried out with heating in dilute mineral acids such as hydrochloric or sulfuric acid, optionally with the addition of glacial acetic acid, dioxane, tetrahydrofuran, or a lower alcohol as a solution promoter. Also useful are perchloric acid or organic acids, such as trifloroacetic, formic, and acetic acid, in combination with catalytic amounts of mineral acids. The alkoxyalkoxymethyl compounds in particular can also be cleaved by using Lewis acids, such as zinc bromide and titanium tetrachloride in anhydrous medium, preferably in dicholoromethane or chloroform, with the 7-bromomethyl or 7-bromozinc derivatives formed as intermediates hydrolyzing spontaneously during the aqueous workup. In the cleavage in mineral acid solution, the reaction temperature must be chosen so that no significant dehydration of the tertiary hydroxyalkyl group in the 1-position occurs; it should therefore be below 100 ° C as a rule.

The reduction of the xanthines of formulas (XI) and (XV) with an oxoalkyl group in the position of $R^5$ or $R^{15}$ to the corresponding hydroxyalkyl compounds can indeed take place in principle either with base metals or by catalytic hydrogenation, but the method of choice consists of the reaction occurring under the very mild conditions and in high yields with simple metal hydrides ($MH_n$), complex metal hydrides ($M^1$-$[M^2H_n]_m$), or organometallic hydrides (Houben-Weyl, Vol. IV/1 d (1981), pp. 267-262, and Vol. VI/1 b (1984), pp. 141-155). Of the numerous complex metal hydrides that can be used for the reduction of ketones, the most frequently used reagents might be mentioned, for example, lithium alanate, lithium borohydride, and especially sodium borohydride, that is easier to handle because of its lower reactivity and above all permits working in alcoholic, alcoholic aqueous, and pure aqueous solutions or suspensions. In addition to the

otherwise customary inert solvents, such as ethers (for example, diethyl ether, tetrahydrofuran, 1,2-dimethoxyethane), hydrocarbons and pyridine, nitriles, such as acetonitrile, can also be used as the reaction medium. The hydrogenation, which is suitably carried out at temperatures between 0 °C and the boiling point of the particular solvent, but preferably at room temperature, generally occurs rapidly and is complete within several minutes to a few hours.

The tertiary hydroxyalkylxanthines of formula (II) can also be prepared by reacting substituted xanthines of formula (XIX)

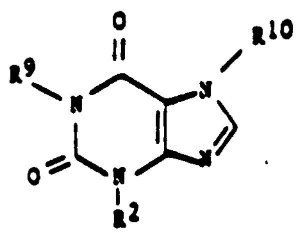

(XIX)

e) contains two identical or different groups of the formula

$-(CH_2)_n-CO-CH_3$ (XX); or

$-(CH_2)_n-CO-R^4$ (XXI),

or only one substituent of the formula (XX) or (XXI), and hydrogen or the group $R^5$ or $R^{15}$ in the positions of $R^9$ and $R^{10}$, with $(C_1-C_3)$alkyl- or methylmetal compounds with reductive "alkylation" of the carbonyl groups to obtain the xanthines pursuant to the invention of formulas (IX) to (XVI), or

f) metallating xanthines of formula (XIX) that have two identical or different groups of the formula $-(CH_2)_n-$Hal (XVII), with Hal preferably standing for chlorine or bromine, or only one such group and hydrogen or the substituent $R^5$ or $R^{15}$ in the other position, in the terminal position, and then reacting them with the ketones of the formula

$R_4-CO-CH_3$ (XVIII)

with reductive alkylation of the carbonyl group to obtain the xanthines of formulas (IX) to (XVI) pursuant to the invention, or

g) coverting xanthines of formula (XIX) with the group

$-(CH_2)_n-COO-(C_1-C_4)$alkyl (XXIV)

in the positions of $R^9$ and/or $R^{10}$ and optionally hydrogen or the group $R^5$ or $R^{15}$ in the other position, by means of two equivalents of a methylmetal compound per alkoxycarbonyl group, into xanthines of formulas (IX) to (XVI) in which $R^4$ stands for methyl, or

h) coverting xanthines of formula (XIX) having two identical or different groups of the formula

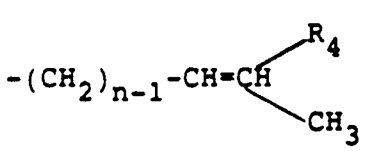

(XXV)

or only one such group and hydrogen or the group $R^5$ or $R^{15}$ in the positions of $R^9$ and $R^{10}$, in which the group (XXV) can contain the C=C double bond also in position-isomeric arrangements on the branched carbon atom, for example, as $-C=CH_2$, by acid-catalyzed hydration obeying the Markownikoff Rule, into the xanthines of formulas (IX) to (XVI) pursuant to the invention, and if desired, then coverting the tertiary hydroxyalkylxanthines of formulas Ib' and if obtained pursuant to the invention by methods e) to h) that have a hydrogen atom in the 1- or 7-position, optionally in the presence of basic media or in the form of their salts, with the alkylating agents of formula (VIII) or (Xa) or (XIII), into the trisubstituted compounds of

formulas (X) or (XI) or (XV), in which $R^2$, $R^4$, $R^5$, $R^{15}$, and n in the formulas above have the meanings indicated above.

The 3-alkylated mono- or dioxoalkyl- (XIXa), -(ω-haloalkyl) (XIXb), -(ω-alkoxycarbonylalkyl)- (XIXc), and -alkenylxanthines (XIXd) needed for this as starting materials are either known or can be prepared readily, for example, from the 3-alkyl-xanthines (VII) and the sulfonyloxy- or haloketones (XVII) and (XVIII), ω-haloalkylsulfonates, or 1,ω-dihaloalkanes (cf., for example: V.B. Kalcheva et al., Journal fur prakt. Chemie 327 (1985) pp. 165-168), ω-sulfonyloxy or ω-halocarboxylic acid alkyl esters or sulfonyloxy or haloalkenes corresponding to formula (XXV) under the reaction conditions previously described in detail for the alkylation of mono- and disubstitued xanthines with the compounds of formulas (VIII) and (Xa).

In the organometallic reactions of the xanthines (XIXa) and (XIXc) functionalized in the $R^9$ and $R^{10}$ groups, the procedure is the same in principle as described for the preparation of the tertiary alcohols of formula (VIII) used as alkylating agents. Thus, the reductive alkylation of the ketones (XIXa) and of the esters (XIXc) can take place, for example, with alkylpotassium, -sodium, -lithium, -magnesium, -zinc, -cadmium, -aluminum, and -tin compounds. The recently recommended alkyltitanium and -zirconium compounds (D. Seebach et al., Agnew, Chem. 95 (1983), pp. 12-26) can also be used. However, since the alkylmetal compounds of sodium and potassium have a tendency toward side reactions because of their high reactivity and those of zinc and cadmium are relatively sluggish, the alkyllithium and -magnesium (Grignard) compounds are ordinarily preferred.

The strong nucleophilic organometallic compounds are very sensitive to hydrolysis and oxidation. Their safe handling therefore requires working in anhydrous medium, optionally under an inert gas atmosphere. The usual solvents or distributing agents are primarily those that are suitable also for the preparation of the alkylmetal compounds. Practical examples are especially ethers with one or more ether oxygen atoms, for example diethyl, dipropyl, dibutyl, or diisoamyl ether, 1,2-dimethoxyethane, tetrahydrofuran, dioxane, tetrahydropyran, furan, and anisole, and aliphatic or aromatic hydrocarbons, such as petroleum ether, cyclohexane, benzene, toluene, xylenes, diethylbenzenes, and tetrahydronaphthalene; however, tertiary amines, such as triethylamine, or dipolar aprotic solvents, such as hexamethylphosphoric triamide, as well as mixtures of the solvents mentioned can also be used successfully. The reaction of the carbonyl compounds (XIXa) and (XIXc) with the Grignard compounds with the formula $R^4$-MgHal can also beneficially be carried out by placing the organometallic compound in an ether and adding the ketone or the ester dropwise as a solution in dischloromethane or 1,2-dicholoroethane. An addition of magnesium bromide is frequently recommended, which is able to increase the nucleophilicity of the organometallic compound because of its participation in the complex cyclic transition state.

The ketone or ester and the organometallic compound are generally combined at temperatures between -20 °C and 100 °C, preferably between 0 °C and 60 °, or at room temperature without external cooling, with the alkylmetal compound ordinarily being used in slight excess. The reaction is then ordinarily completed by brief heating under reflux, for which times of several minutes to a few hours are generally adequate. The alkanolate formed is preferably decomposed with aqueous ammonium chloride solution or dilute acetic acid.

Metallic magnesium and lithium are primarily suitable for the metallation of the ω-haloalkylxanthines (XIXb). On the other hand, the replacement of the halogen atom with lithium, which is also possible using organolithium reagents, generally 1-butyl-, 2-butyl-, t-butyl-, or phenyllithium, plays a subordinate role. However, use is made especially of the Grignard compounds, advantageously preparing them in the ethers, hydrocarbons, tertiary amines, or aprotic solvents listed as particularly suitable for the reaction of the xanthines (XIXa) and (XIXc) with alkylmetal compounds, at temperatures between 25 ° and 125 °C, preferably below 100 °C. If the metallation reaction is carried out in hydrocarbons, then the addition of an ether, such as tetrahydrofuran, or a tertiary amine, such as triethylamine, in stoichiometric amount frequently proves useful. The use of catalysts, such as butanol, aluminum chloride, silicon tetrachloride, tetrachloromethane, and aluminum or magnesium alcoholates, may also be helpful. In the halogen-metal exchange the chlorides ordinarily react more slowly than the corresponding bromides and iodides, but as a rule they provide better yields of organometallic compound. To accelerate the beginning of the reaction, the addition of some magnesium bromide, some grains of iodine, or several drops of bromine, tetrachloromethane, or methyl iodide with slight heating is frequently recommended. The Grignard compounds obtained are normally not isolated, but are reacted immediately with the ketones of formula (XXIII) under the reaction conditions described for the reductive alkylation of the xanthines (XIXa) and (XIXc).

The addition of water to the C=C double bond of the alkenylxanthines (XIXd) with the structural element of formula (XXV), in which the hydroxy group adds to the carbon atom with the fewer hydrogens to form tertiary alcohols according to the Markownikoff Rule, ordinarily occurs in aqueous solution or suspension in the presence of strong acids, such as sulfuric, nitric, or phosphoric acid. Hydrogen halides and sulfonic acids, such as trifluoromethanesulfonic acid, acid exchange resins, boron trifluoride complexes,

or oxalic acid, can also be used as catalysts. However, it is preferred to operate in sulfuric acid, with an acid concentration of 50 to 65% and temperatures of 0° to 10°C being sufficient as a rule. However, lower or higher acid concentration and/or reaction temperatures can sometimes also be used. In any case, the reaction temperatures should be kept as low as possible since the reverse dehydration to the olefin can be disturbingly significant above approximately 60°C.

The addition of a solvent inert to acids, such as 1,4-dioxane, benzene, or toluene, sometimes also provides benefits. Since esters can form as intermediates in the acid-catalyzed hydration, particularly when using the high acid concentrations, it is recommended to treat the reaction batch with a large amount of water with brief heating after the action of the acid for the purpose of ester hydrolysis, or to process the mixture in the alkaline range.

The experimental conditions for the optional conversion of the 1- and 7H-compounds (IX) or (XVI) pursuant to the invention into the trisubstituted xanthines of formulas (X) or (XV) by N-alkylation with the compounds (VIII) or (Xa) of (XIII) have already been described above in detail.

Depending on the chain length of the alkyl group $R^4$ (at least $C_2$) and/or the structure of a substituent $R^5$ (for example, 2-hydroxypropyl), the tertiary hydroxyalklyxanthines of formula (II) can have one or two asymmetric carbon atoms and can thus be present in stereoisomeric forms. This invention therefore concerns both the pure stereoisomeric compounds and their mixtures.

This invention will now be described in greater detail in the following Examples. More particularly, to demonstrate the effectiveness of the claimed invention, a compound of formula (II) was tested to demonstrate inhibition of the effects of GVHR *in vivo*. Though a variety of compounds within the general formulas (I) and (II) are effective, they will be exemplified with regard to pentoxifylline ("PTX") as a particularly preferred xanthine.

## EXAMPLE 1

### Tolerancce of Bone Marrow Transplant Patients for Pentoxifylline

Study Design. Dose escalation trial in which groups of 10 consecutive patients were enrolled at increasing dose levels. Pentoxifylline was given at three doses: 1200 mg, 1600 mg, and 2000 mg/day, orally as 3, 4, and 5 divided daily doses respectively from day -10 through day +100 posttransplant. Pills were crushed and mixed with liquid for patients who experienced difficulty swallowing intact caplets. Vomited doses were repeated if vomiting occurred within 30 minutes of administration of a crushed dose or if an intact pill was recovered.

Patients. Thirty (30) consecutive patients were enrolled in this study and included autologous, matched and mismatched allogeneic transplants. Because transplant-related toxicities vary among the type of transplant, only patients with phenotypic or genotypically identical donors were included in this analysis. Of the 30 study patients enrolled, data from 18 matched allograft recipients (mean age 34.6 years), who were ≧ 100 days posttransplant, were analyzed. Fifteen patients received HLA-identical sibling transplants while 3 patients underwent matched transplants from unrelated donors. Diagnosis and disease phase are shown in Table I.

Table I

Characteristics of Patients

Group I

| UPN¶ | Age | Sex' | Disease¥ | Disease State* | Preparative Regimen§ | HLA Identity● | GM CSF* | PTX Dose' |
|---|---|---|---|---|---|---|---|---|
| 5299 | 44 | M | PLL | Rel | Cy/1320 | M | Yes | 1600 |
| 5420 | 37 | M | NHL | Rel | Cy/1440 | M | Yes | 1600 |
| 5456 | 41 | M | NHL | Rel | CBV | M | Yes | 1600 |
| 5518 | 36 | M | MM | Rem | Bu/Cy | M | Yes | 1600 |
| 4791 | 41 | M | NHL | Rel | Cy/1200 | M | No | 1600 |
| 5552 | 23 | M | AML | Rem | Cy/1200 | M | No | 1600 |
| 5635 | 33 | M | CML | AP | Bu/Cy/1200 | M | No | 2000 |
| 5715 | 22 | M | ALL | Rel | Cy/1440 | M | No | 2000 |

Group II

| UPN¶ | Age | Sex' | Disease¥ | Disease State* | Preparative Regimen§ | HLA Identity● | GM CSF* | PTX Dose' |
|---|---|---|---|---|---|---|---|---|
| 5300 | 42 | M | MM | Rel | Bu/Cy | M | Yes | 1200 |
| 5448 | 23 | M | ALL | Rem | Cy/1440 | M | Yes | 1200 |
| 5265 | 23 | M | ALL | Rel | Cy/1440 | M | Yes | 1200 |
| 5517 | 24 | M | CML | CP | Cy/1200 | URD | Yes | 1200 |
| 5549 | 37 | M | CML | CP | Cy/1200 | URD | Yes | 1200 |
| 5705 | 46 | M | CML | AP | Cy/1320 | URD | Yes | 2000 |
| 5360 | 42 | M | CML | CP | Cy/1200 | M | No | 1200 |
| 5362 | 41 | M | CML | CP | Cy/1200 | M | No | 1200 |
| 5554 | 43 | M | CML | CP | Cy/1200 | M | No | 1600 |
| 5653 | 26 | M | CML | CP | Cy/1200 | M | No | 1600 |

¶ - Unique Patient Number
' M - Male
¥ ALL - Acute Lymphocytic Leukemia
AML - Acute Myelogenous Leukemia
NHL - Non Hodgkin's Lymphoma
MM - Multiple Myeloma
CML - Chronic Myelogenous Leukemia
PLL - Prolymphocytic Leukemia

* Rel - Relapse or Refractory disease
Rem - 2nd or 3rd remission
CP - Chronic Phase
AP - Accelerated Phase

§ Bu - Busulfan
Cy - Cyclophosphamide
1440 - (cGy) Total Body Irradiation
CBV - Cytoxan, Carmustine, VP-16

' PTX - Pentoxifylline(mg/day)
*GM-CSF - 250ug/M2/day i.v. days 0-21

● M - related HLA-identical donor
URD - matched unrelated donor

Prophylaxis and treatment of acute GVHD. Prophylaxis of aGVHD consisted of either cyclosporine (CSP) plus methylprednisolone (MP) or cyclosporine plus short methotrexate (MTX) [13,14]. The pretransplant characteristics and preparative regimens are listed in Table I. Patients were grouped for analysis on the basis of whether they received MP (Group I, n = 8) or MTX (Group II, n = 10) along with CSP as their aGVHD prophylaxis. This was done because previous studies suggest slower engraftment and more mucositis with MTX containing GVHD regiments. Acute GVHD was diagnosed and graded according to previously published criteria [15] and was treated, in both groups, with methylprednisolone (2 mg/kg/day for three weeks).

Posttransplant Supported Care. All patients were nursed in single reverse isolation rooms and received intravenous hyperalimentation and *Pneumocystis carinii* prophylaxis (trimethoprim-sulfamethoxazole) along with monthly intravenous immune globulin at a dose of 500 mg/kg (Sandoglobulin™ Sandoz, NJ). Patients were maintained on parenteral nutrition until they were capable of sustaining 100% of their calculated fluid and caloric requirements orally. Seventeen patients were CMV seropositive pretransplant and received prophylactic high dose acyclovir daily until time of discharge [16] at which time oral acyclovir (400 mg

q.i.d.) was administered until day +75 posttransplant. All patients received prophylactic systemic antibiotics consisting of cefotaxime and mezlocillin if febrile (T≧ 38.5 ° C) or when their absolute neutrophil count (ANC) was ≦500 cells/µL and continued until ANC rose ≧500 cells/µL. Patients with unexplained fever for ≧48 hours received presumptive anti-fungal therapy with amphotericin B (0.3 g/kg i.v.) until resolution of neutropenia. Weekly rapid diagnostic cytomegalovirus (CMV) shell-vial assays were routinely performed beginning day +35 on buffy coat and urine samples in all CMV seropositive patients. Patients excreting CMV were treated with a two week course of DHPG (10 mg/kg/day i.v. Ganciclovir™), followed by an additional four weeks of five times weekly maintenance therapy (5 mg/kg/day). In addition, 10 patients received recombinant human GM-CSF (rhGM-CSF) at a dose of 250 µg/M$^2$/day i.v. over 2 hours, days 0-21 (Immunex, Seattle).

Toxicity Definitions. Acute toxicity was judged according to published criteria [17]. Renal insufficiency was defined as a doubling of the baseline (day 0) serum creatinine (Scr); renal failure was defined as a Scr ≧3.0 mg/dL with a blood urea nitrogen (BUN) ≧ 80 mg/dL. The diagnosis of hepatic VOD required the presence of hyperbilirubinemia (≧ 3.0 mg/dL), weight gain ≧ 2.5% of baseline weight and right upper quadrant pain, with or without the presence of hepatomegaly [18]. Severity of mucositis was scored in accordance with previous published criteria [14]. In this analysis ≧ grade II severity was considered a toxicity. The number of days of continuous morphine sulfate ($MSO_4$) administration was used as an objective parameter for severity of mucositis.

PTX Toxicity. No patient experienced significant adverse side effects at any of the dose levels administered. Mild gastrointestinal symptoms occurred in 2 patients, both relieved by administration of oral antacids.

Survival. With a mean follow-up of 160 days (range 100-320) there were no regimen related deaths. One patient died of relapse on day 132.

Transplant Related Toxicities. One patient in Group I and one Group II patient (11%) developed mild hepatic VOD with neither patient experiencing more than 2.5% increase in basal body weight during the first 21 days. Only 1 patient developed renal insufficiency. One patient received 4.5 grams amphotericin B pretransplant for hepato-splenic candidiasis and received an additional 1.0 grams of amphotericin B prophylactically during the first 30 days of his transplant course. No patient in Group I experienced mucositis of significant severity to require $MSO_4$, whereas 5 (50%) of Group II patients required $MSO_4$. Patients receiving PTX doses in excess of 1200 mg/day had less significant mucositis with only 1 patient requiring analgesia in contrast to 5 to 7 patients who received the 1200 mg/day dose. All patients in Group I were able to take 100% of their daily caloric requirements orally by a mean of day 18, with Group II Patients achieving 100% of their caloric requirements orally by a mean of day 29.6. The mean time to discharge for both groups was day 26.4.

GVHD. The incidence of GVHD was low in both groups. Overall, 78% had grade 0-I GVHD (Table II). One patient (12.5%) in Group I developed grade II GVHD involving predominantly skin, while 3 patients (30%) in Group II, all of who received PTX at the lowest dose level, experienced grade II GVHD; all episodes-resolved with steroid treatment. No patient developed severe (i.e. ≧ grade III) GVHD.

Table II

Outcome of Patients

**Group I**

| UPN¶ | Day ANC' ≥ 500/uL | No Days T°≥38.3°C Day 0-28 | Maximum Bilirubin Day 0-21 | Maximum Creatinine Day 0-28 | Days $MSO_4$¥ required | GVHD§ Grade | Last Day of TPN⁻ | Day of Discharge | Day (Status) of last Contact• | GM-CSI |
|---|---|---|---|---|---|---|---|---|---|---|
| 5299 | 20 | 0 | 4.7 | 1.0 | 0 | II | 20 | 22 | 320(+) | Y |
| 5420 | 11 | 0 | 1.2 | 1.2 | 0 | 0 | 18 | 18 | 200(+) | E |
| 5456 | 12 | 0 | 1.1 | 1.1 | 0 | 0 | 17 | 16 | 160(+) | S |
| 5518 | 14 | 1 | 2.3 | 1.1 | 0 | 0 | 15 | 15 | 130(+) | |
| 4791 | 16 | 0 | 1.6 | 0.8 | 0 | 0 | 18 | 18 | 120(+) | |
| 5552 | 22 | 0 | 1.9 | 1.2 | 0 | 0 | 26 | 24 | 100(+) | N |
| 5635 | 25 | 4 | 2.3 | 1.0 | 0 | 0 | 20 | 26 | 120(+) | O |
| 5715 | 12 | 7 | 1.2 | 1.7 | 0 | 0 | 15 | 16 | 100(+) | |
| Mean ± SEM | 16.5 ± 1.8 | 1.5 ± 0.9 | 2.0 ± 0.4 | 1.1 ± 0.09 | 0 | | 19.1 ± 1.3 | 19.8 ± 1.5 | | |

**Group II**

| UPN¶ | Day ANC' ≥ 500/uL | No Days T°≥38.3°C Day 0-28 | Maximum Bilirubin Day 0-21 | Maximum Creatinine Day 0-28 | Days $MSO_4$¥ required | GVHD§ Grade | Last Day of TPN⁻ | Day of Discharge | Day (Status) of last Contact• | GM-CSI |
|---|---|---|---|---|---|---|---|---|---|---|
| 5300 | 23 | 0 | 1.4 | 1.1 | 0 | II | 27 | 31 | 250(+) | |
| 5448 | 18 | 6 | 2.7 | 0.9 | 13 | 0 | 35 | 36 | 190(+) | Y |
| 5265 | 26 | 7 | 1.5 | 1.3 | 20 | II | 35 | 39 | 132 R* | E |
| 5517 | 32 | 3 | 4.3 | 1.4 | 0 | I | 39 | 40 | 140(+) | S |
| 5549 | 23 | 2 | 0.9 | 0.7 | 0 | II | 32 | 35 | 150(+) | |
| 5705 | 18 | 6 | 2.6 | 1.4 | 10 | 0 | 19 | 20 | 100(+) | |
| 5360 | 22 | 6 | 3.1 | 1.3 | 14 | I | 25 | 26 | 210(+) | |
| 5362 | 25 | 7 | 7.4 | 1.0 | 15 | 0 | 28 | 31 | 220(+) | N |
| 5554 | 21 | 2 | 1.7 | 0.8 | 0 | 0 | 25 | 25 | 150(+) | O |
| 5653 | 19 | 0 | 1.8 | 0.8 | 0 | 0 | 21 | 21 | 110(+) | |
| Mean ± SEM | 22.7 ± 1.3 | 3.9 ± 0.8 | 2.7 ± 0.6 | 1.07 ± 0.08 | 7.2 ± 2.5 | | 29.6 ± 1.9 | 31.5 ± 2.1 | | |

¶ UPN - Unique Patient Number
•(+) - still alive
§ GVHD - graft-versus-host disease
¥MSO4 - morphine sulfate
°T - Temperature
⁻ TPN - Total parenteral nutrition
R* - Relapse
' ANC - Absolute neutrophil count

Fever and Infection. The overall incidence of fever (Temp ≧ 38.5 ° C) was 60% with 11 patients experiencing fever for a mean of 4.6 days. Patients in Group I had fewer febrile days than those in Group II. Only 1 (5.5%) patient had a positive blood culture within the initial hospitalization period. One patient had a coagulase negative staphylococcus isolated on day 2 posttransplant. Sixteen of 17 CMV seropositive patients (94%) had evidence of CMV excretion during the first 100 days posttransplant. No patient

developed evidence of CMV tissue infection.

Engraftment and marrow function. The outcome of patients who were treated with the two different GVHD prophylactic regimens are shown in Table II. All patients completely engrafted and no episodes of graft rejection were seen. Whether or not they received GM-CSF, patients who did not receive MTX (Group I) as part of their GVHD prophylaxis had accelerated neutrophil recovery when compared to patients receiving MTX (Group II).

The purpose of this study was to first determine the tolerability of PTX in BMT patients and second, to estimate if PTX might have an effect on the expression of post-BMT toxicities. Eighty-eight percent of patients were considered high risk for transplant related toxicities by virtue of age [22], disease phase, and preparative regimen [17]. All patients tolerated oral PTX with >95% of prescribed doses taken with no toxicity noted. In addition, there was a strong suggestion that PTX ameliorated transplant related toxicities. Only 2 patients developed mild VOD associated with minimal weight gain not requiring therapy and only one patient experienced mild renal insufficiency. This is in contrast to the recent reported incidence of 57% and 53% for VOD and renal insufficiency, respectively [1,18]. In addition no episodes of interstitial pneumonitis, either idiopathic or due to CMV, were seen in the first 100 days; the latter likely the result of early treatment of CMV excretion with anti-viral therapy. Similarly, the incidence of mucositis was low in both groups [13].

As anticipated GM-CSF appeared to accelerate neutrophil recovery in patients receiving MP (Group I) and may have contributed to the reduction in febrile days observed between the two groups. However, compared to other patients who received GVHD prophylaxis with CSP + MP but without PTX [23], 75% of patients receiving GM-CSF alone experienced fever for a mean of 7 days (range 1-19) compared to 37% of our patients receiving PTX ± GM-CSF (mean 4 days, range 1-7); suggesting PTX may act synergistically with GM-CSF on modulating inflammatory mediators. The overall incidence of grade II aGVHD was also low being just 22%, predominantly involving skin. No patient developed multi-system aGVHD and only 1 patient developed mild, isolated chronic hepatic GVHD on a follow-up.

In this study the oral administration of PTX up to doses of 2000 mg/day appeared to be well tolerated with no adverse side effects observed. Unlike other methylxanthine derivatives, PTX lacks much of the cardiovascular side effects; a finding supported by this phase I-II study.

Compared to recent historical control patients, PTX appeared to reduce morbidity and mortality in patients undergoing allogeneic BMT. If, in fact, PTX does ameliorate transplant-related toxicities without an increase in relapse rates, then the use of PTX alone or in combination with cytokines like GM-CSF may permit the delivery of higher, more effective doses of chemoradiotherapy resulting in lower relapse rates and improved overall event free survival.

## EXAMPLE 2

### Effect of Pentoxifylline On Regimen Related Toxicity In Patients Undergoing Bone Marrow Transplantation

Recurrent disease and regimen related toxicity ("RRT") accounts for 25-75% of treatment failures in patients undergoing bone marrow transplantation ("BMT"). Lower relapse rates observed with more intensive chemoradiation regimens have been offset by increases in regimen related deaths. A pilot study of prophylactic oral PTX, (400 mg q.i.d. until day 100 post BMT), in 8 consecutive allograft recipients, was conducted. Six patients received marrow from HLA-identical siblings, while 2 received related grafts that differed at 1 HLA-locus. All patients were prepared with a combination of cytoxan and hyperfractionated irradiation. When compared to 20 age, disease, regimen and HLA matched controls, PTX significantly reduced the incidence and severity of all major complications post BMT as shown below.

| Complication | Controls n=20 | PTX n-8 | p* |
|---|---|---|---|
| Mean age (years) | 31.6 ± 1.5 | 34.9 ± 2.1 | 0.51 |
| Mucositis (days MSO4) | 18.7 ± 1.1 | 4.4 ± 2.2 | 0.007 |
| Incidence of Hepatic Venooclusive Disease | 65% | 25% | 0.04 |
| Renal Insufficiency (doubling of day 0 creatinine) | 65% | 0% | 0.009 |
| Incidence hypertension (blood pressure ≥ 140/95) | 95% | 0% | 0.005 |
| Incidence of Graft vs. Host Disease | | | |
| ≤ Grade I | 45% | 100% | 0.045 |
| ≥ Grade II | 55% | 0% | 0.005 |

*Wilcoxson signed rank analysis. All values mean ± SEM

There were no adverse side effects associated with PTX administration, with the majority of patients >100 days posttransplant without evidence of recurrent disease.

In summary, the use of xanthines according to this invention makes it possible to increase the feasibility of transplant procedures. The xanthines are valuable therapeutic agents for both prophylaxis and treatment of allograft reaction, such as HVGR and GVHD. Survival following engraftment is primarily related to the severity of acute GVHD. This invention makes it possible to inhibit GVHR, and a related reduction in GVHD is anticipated. As a result, the prognosis of transplant patients should improve. As the method of this invention is a new procedure affecting the prognosis of cell, tissue, and organ transplantation, the requirements for donor and recipient histocompatibility may change and thus also the criteria for donor selection.

PUBLICATIONS CITED IN SPECIFICATION

1. Zager R.A., O'Quigley J., Zager B.K. et al. Acute renal failure following bone marrow transplantation. A retrospective study of 272 patients. Am J Kidney Diseases 1989.

13. Storb R., Deeg H.J., Whitehead, J. et al. Methotrexate and cyclosporine compared with cyclosporine alone for prophylaxis of acute graft vs. host disease after marrow transplantation for leukemia. N Eng J Med 1986; 314:729-735.

14. Tutschka P.J, Copelan E.A., Klein J.P. Bone marrow transplantation following a new busulfan and cyclophosphamide regimen. Blood 1987; 5:1382-1388.

15. Glucksberg H, Storb R, Fefer A., et al. Clinical manifestations of graft v. host disease in human recipients of marrow from HLA-matched sibling donors. Transplantation 1974; 18:295-304.

16. Meyers J.D., Reed E.C., Shepp D.H., et al. Acyclovir for prevention of cytomegalovirus infection and disease after allogeneic marrow transplantation. N Eng J Med 1988 1988; 318:70-75.

17. Bearman S.I., Appelbaum F.R., Buckner C.D., et al. Regimen related toxicity in patients undergoing bone marrow transplantation. J Clin Oncol 1988; 6:1562-68.

18. McDonald G.B., Sharma P., Matthews D.E., et al. Venocclusive disease of the liver after bone marrow transplantation: Diagnosis, incidence and predisposing factors. Hepatology 1984; 4:116-122.

19. Bevilacqua M.P., Pober J.S., Majeau G.R., et al. Recombinant tumor necrosis factor alpha induces procoagulant activity in cultured human vascular endothelium. Characterization and comparison with the actions of interleukin-1. Proc Nat'l Acad Sci USA 1986; 83:4533-37.

20. Nawroth P.P, Stern D.M., Modulation of endothelial hemostatic properties by tumor necrosis factor alpha. J Exp Med 1986; 163:740-45.

21. Detmar M., Tenono S., Imcke E., et al. TNF alpha but not IL-6 induces HLA-DR and ICAM-1 expression in culture derma microvascular endothelial cells. Anti-TNF monoclonal antibody inhibits this

induction Clin Res 1990; 38:646A.
22. Klingeman H.G., Storb R., Fefer A., et al. Bone marrow transplantation in patients aged 45 years and older. Blood 1986; 67:770.
23. Nemunaitis J., Appelbaum, F.R., Higano C.S. et al., Phase I/II trial of recombinant human granulocytemacrophage colony stimulating factor following allogeneic bone marrow transplantation. Blood (in press).
24. Sinzinger H., Firbar W., Irradiation depresses prostacyclin generation upon stimulation with platelet derived growth factor. Br J Radiol 1985; 58:1023-26.
25. Nash R., Pepe N., Storb R., et al. Cyclosporine (CSP) and methotrexate (MTX) prophylaxis of acute GVHD: Analysis of risk factors, [abstract] Blood 1990 (in press).
26. Duffie G.P., Romanelli R.R., Ellis N.K., et al. The effects of E series prostaglandins on blastogenic responses in vitro and graft vs. host responses in vivo. Immunopharmacol Immunotoxicol 1988; 10:597-615.
27. Wiederkehr J.C., Dumble L., Pollak R., et al. Immunosuppressive effect of misoprostil: a synthetic prostaglandin E1 analogue. Aust N Z J Surg 1990; 60:121-4.
28. Perl A., Lang I., Gonzalez-Cabello R., et al. Indomethicin abrogates the suppression by cyclosporine A of lectin-dependent cell-mediated cytotoxicity to HEp-2 cells Immunopharmacology 1986; 11:39-45.

## Claims

**1.** Use of at least one 7-(oxoalkyl) 1,3-dialkyl xanthine, other than denbufylline, of the formula I:

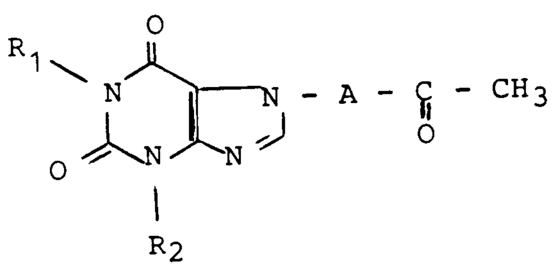

(I)

in which
$R_1$ and $R_2$ are the same or different and are selected from the group consisting of straight-chain or branched alkyl radicals with 2 to 6 carbon atoms, cyclohexyl, straight-chain or branched chain alkoxyalkyl and hydroxyalkyl radicals;
A represents a hydrocarbon radical with up to 4 carbon atoms, which can be substituted by a methyl group;
for the preparation of a medicament for inhibiting allograft reaction in human.

**2.** Use of a compound as defined in claim 1 for the preparation of a medicament for inhibiting allograft reaction in human, wherein said allograft reaction is host versus graft reaction or graft versus host reaction.

**3.** Use of recombinant human GM-CSF for the preparation of a medicament for inhibiting allograft reaction in human.

**4.** Use of at least one compound of the formula II:

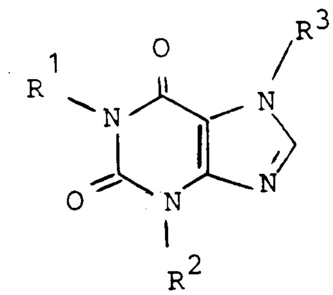

(II)

wherein at least one of $R^1$ and $R^3$ is either

a) a branched hydroxyalkyl group of the formula

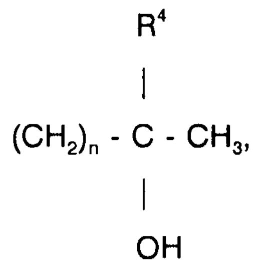

in which $R^4$ stands for an alkyl group with 1 to 3 carbon atoms and n stands for a whole number from 2 to 5, the other $R^1$ or $R^3$ group that may optionally be present stands for a hydrogen atom or an aliphatic hydrocarbon group $R^5$ with up to 6 carbon atoms, whose carbon chain may be interrupted by up to 2 oxygen atoms or may be substituted with a hydroxy or oxo group, or

b) an oxoallyl group of the formula

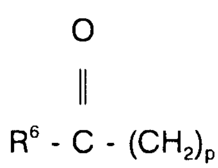

wherein $R^6$ is $C_1$-$C_6$ and p is 2, 3 or 4, the remaining $R^1$ or $R^3$ being as defined above, and $R^2$ is an alkyl group $C_1$-$C_4$; for the preparation of a medicament for inhibiting allograft reaction in human.

**5.** Use of a compound as defined in claim 4 for the preparation of a medicament for inhibiting allograft reaction in human, wherein said allograft reaction is host versus graft reaction or graft versus host reaction.

**6.** Use of a compound as defined in claim 4, wherein said compound is pentoxifylline.

**7.** Use of a compound as defined in claim 4, wherein said compound of formula II

$R^1$ is

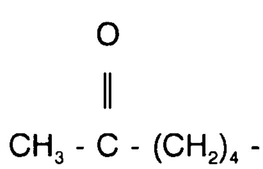

$R^2$ is $-CH_3$

and

$R^3$ is $-CH_2-CH_2-CH_3$.

**8.** Use of a compound as defined in claim 4, wherein said compound of formula II
$R^1$ is

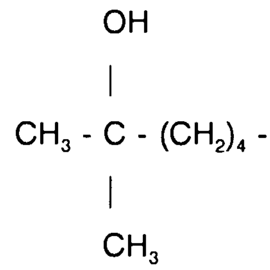

$R^2$ is $-CH_3$
and
$R^3$ is $-CH_3-CH_2-O-CH_3$.

**9.** Use of a compound as defined in claim 4, wherein said compound of formula II
$R^1$ is

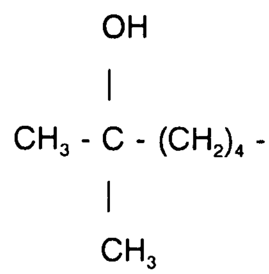

$R^2$ is $-CH_3$
and
$R^3$ is $-CH_2-O-(CH_2)_2-O-CH_3$.

**10.** Use of a compound as defined in claim 4, wherein said compound of formula II
$R^1$ is

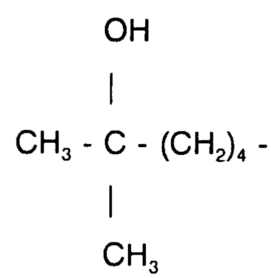

$R^2$ is $-CH_3$
and
$R^3$ is -H.

**11.** Use of a compound as defined in claim 4, wherein said compound of formula II
$R^1$ is

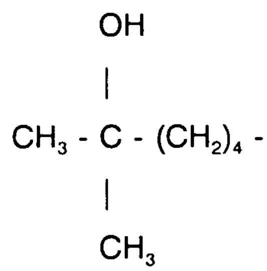

$R^2$ is $-CH_3$
and
$R^3$ is $-CH_2 - CH_2 - CH_3$.

**12.** Use of a compound as defined in claim 4, wherein said compound of formula II
$R^1$ is

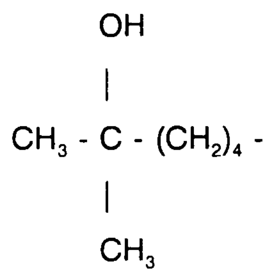

$R^2$ is $-CH_3$
and
$R^3$ is

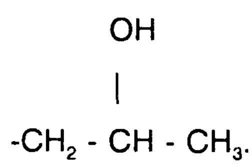

**13.** Use of a compound as defined in claim 4, wherein said compound of formula II
$R^1$ is

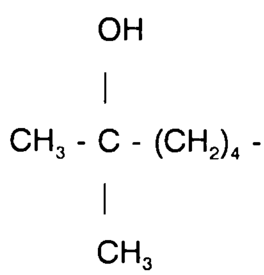

$R^2$ is $-CH_3$
and
$R^3$ is

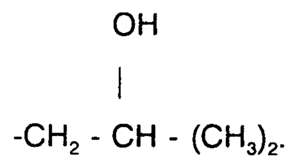

**14.** Use of a compound as defined in claim 4, wherein said compound of formula II
$R^1$ is

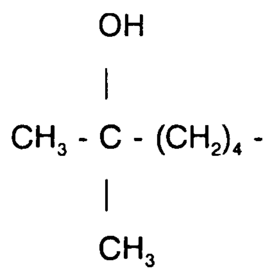

$R^2$ is $-CH_2-CH_3$
and
$R^3$ is $-CH_2-O-CH_2-CH_3$.

**15.** Use of a compound as defined in claim 4, wherein said compound of formula II
$R^1$ is

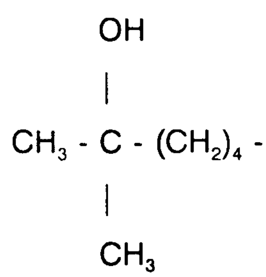

$R^2$ is $-CH_3$
and
$R^3$ is

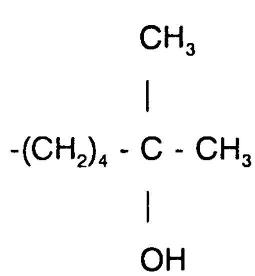

**16.** Use of a compound as defined in claim 4, wherein said compound of formula II
$R^1$ is

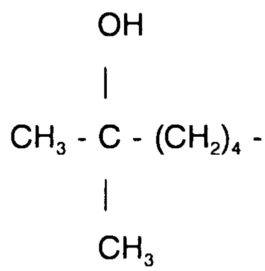

$R^2$ is $-CH_3$

and

$R^3$ is $-CH_2-O-CH_2-CH_3$.

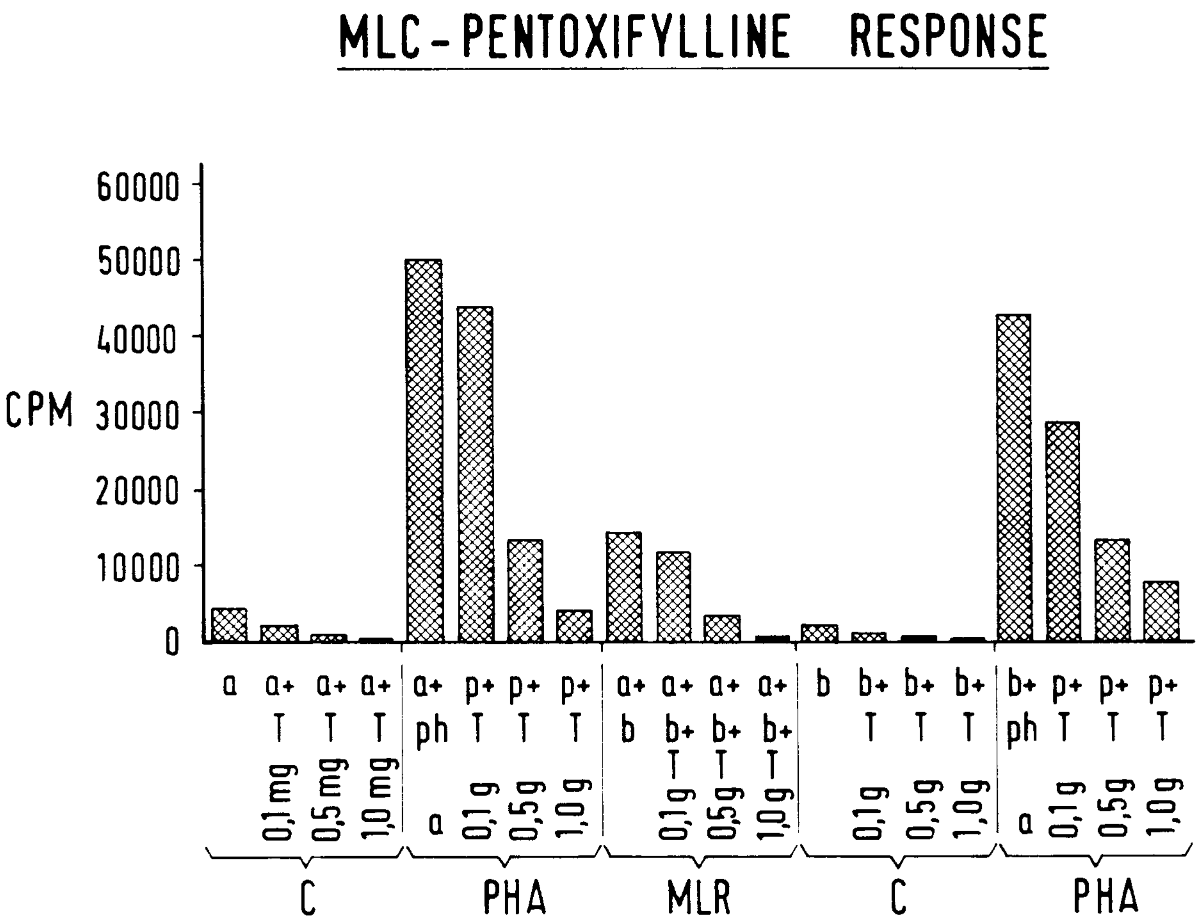
MLC-PENTOXIFYLLINE RESPONSE
CPM
60000
50000
40000
30000
20000
10000
0
a
a+ T 0,1 mg
a+ T 0,5 mg
a+ T 1,0 mg
a+ ph a
p+ T 0,1 g
p+ T 0,5 g
p+ T 1,0 g
a+ b
a+ b+ T 0,1 g
a+ b+ T 0,5 g
a+ b+ T 1,0 g
b
b+ T 0,1 g
b+ T 0,5 g
b+ T 1,0 g
b+ ph a
p+ T 0,1 g
p+ T 0,5 g
p+ T 1,0 g
C
PHA
MLR
C
PHA